# EUROPEAN PATENT SPECIFICATION

(11) **EP 4 058 805 B1**
(45) Date of publication and mention of the grant of the patent: **02.10.2024**
(21) Application number: 20811699.6
(22) Date of filing: 10.11.2020
(51) Int. Cl.: G01N 33/574

(54) **ANTI-CLEVER-1 AGENTS FOR CONTROLLING THE EXPRESSION OF CELL SURFACE MARKERS ON LEUCOCYTES, AND USING THESE TO GUIDE ANTI-CLEVER-1 BASED CANCER TREATMENT**
ANTI-CLEVER-1-WIRKSTOFFE ZUR KONTROLLE DER EXPRESSION VON ZELLOBERFLÄCHENMARKERN AUF LEUKOZYTEN UND VERWENDUNG DIESER MITTEL ZUR ANLEITUNG EINER KREBSBEHANDLUNG AUF ANTI-CLEVER-1-BASIS
AGENTS ANTI-CLEVER-1 POUR RÉGULER L'EXPRESSION DE MARQUEURS DE SURFACE CELLULAIRE SUR DES LEUCOCYTES, ET UTILISATION DE CEUX-CI POUR GUIDER UN TRAITEMENT DU CANCER À BASE D'ANTI-CLEVER-1

(30) Priority: 11.11.2019 FI 20195959
(43) Date of publication of application: 21.09.2022
(73) Proprietor: Faron Pharmaceuticals OY, 20520 Turku (FI)
(72) Inventor: HOLLMÉN, Maija-Leena, 20760 PIISPANRISTI (FI); VIRTAKOIVU, Reetta, 20810 TURKU (FI); VAURA, Felix, 20100 TURKU (FI); JALKANEN, Juho, 23120 MIETOINEN (FI); MANDELIN, Jami, 00730 HELSINKI (FI)
(74) Representative: Berggren Oy
(86) International application number: PCT/FI2020/050741
(87) International publication number: WO 2021/094652

(56) References cited:
- WO-A1-2017/182706
- WO-A2-03/057130
- VIITALA MIRO ET AL: "Immunotherapeutic Blockade of Macrophage Clever-1 Reactivates the CD8 + T-cell Response against Immunosuppressive Tumors", CLINICAL CANCER RESEARCH, AMERICAN ASSOCIATION FOR CANCER RESEARCH, US, vol. 25, no. 11, 1 June 2019 (2019-06-01), pages 3289 - 3303, XP009515480, ISSN: 1078-0432, DOI: 10.1158/1078-0432.CCR-18-3016

## Description

### Field of the invention

The present invention relates to monitoring expression levels of two, three or more cell surface marker in circulating T cell populations in relation to anti-Clever-1 treatment and choosing the best combination agent to initiate treatment together with anti-Clever-1 therapy after the observed changes in the expression of a cell surface marker.

### Background of the invention

The vast number of genetic and epigenetic changes that are inherent to cancer cells provide plenty of tumor-associated antigens that the host immune system can recognize, thereby requiring tumors to develop specific immune resistance mechanisms. An important immune resistance mechanism involves immune-inhibitory pathways, termed immune checkpoints, which normally mediate immune tolerance and mitigate collateral tissue damage. A particularly important immune-checkpoint receptor is cytotoxic T-lymphocyte associated antigen 4 (CTLA-4), which downmodulates the amplitude of T cell activation. Antibody blockade of CTLA-4 in mouse models of cancer induces antitumor immunity. Some immune-checkpoint receptors, such as programmed cell death protein 1 (PD-1), limit T cell effector functions within tissues. By upregulating ligands for PD-1, tumor cells block antitumor immune responses in the tumor microenvironment. [1] Anti-PD-1, anti-PD-L1 and anti-CTLA-4 immune-checkpoint inhibitors are extensively used in clinical patient care. The second generation immune-checkpoint receptors including but not limited to ICOS (Inducible T-cell COStimulator), OX40, 41 BB, TIM3 and LAG3, and antagonist antibodies against these checkpoint inhibitors (ICIs) are under clinical development as anti-cancer agents.

Currently, immune checkpoint modulators targeting CTLA-4 and the PD-1/PD-L1 axis are approved for clinical use, and while highly efficacious in about 10-20 % of patients with melanoma and certain other tumors, several other important cancer types (such as prostate, breast and colorectal cancer) remain refractory to them, and there is no clear biomarker available that could differentiate responders from non-responders and guide treatment [2]. Patients responding favorably to checkpoint inhibition usually have a preexisting antitumor immune response, which is characterized by high density of interferon gamma (IFNg) producing CD8⁺ T cells, expression of PD-L1 in tumor-infiltrating immune cells, and high mutational load. Tumors that do not respond to immune checkpoint blockage show either a stromal T cell phenotype where infiltration of T cells (TIL) into tumors or activation of T cells in the tumor microenvironment (TME) is prevented by immunosuppressive stromal compartments, or a non-inflamed phenotype characterized by low T cell infiltration, low mutational load and high proliferation of tumor cells. The tumors can be classified immunologically (inflamed vs. non-inflamed) based on the presence of tumor infiltrating cytotoxic CD8 T cells [3]. The inflamed tumors show high mutational load, high IFNg and PD-L1 expression, and respond favorably to immune checkpoint blocking therapies. IFNg produced by T cells is considered necessary for ICIs to work as anti-cancer agents. IFNg secretion however, is known to increase PD-L1 expression on cells, which can be a source of immunotherapy resistance [3].

Innate immune cells such as macrophages, however, can dampen T cell activation and contribute to tumor progression despite high mutational load. The macrophages that contribute to tumor-related immunosuppression and provide tumor growth supporting signals may be highly eligible candidates for targeted therapies, since these cells are abundantly present in various tumors, they are very plastic and can be converted into pro-inflammatory macrophages supporting T cell activation and tumor rejection [4, 5]. To date, macrophage targeted strategies under clinical development utilize macrophage colony-stimulating factor receptor inhibition to deplete macrophage populations in tumors [6]. However, resistances to these approaches have already been reported [7]. Thus, there is a need to find novel ways to utilize these cells to induce tumor cell killing by the immune system.

In recent years, increasing attention has been paid to the contribution of scavenger receptors in regulating macrophage responses to different stimuli. Clever-1 (also known as Stabilin-1) is a multifunctional molecule conferring scavenging ability on a subset of anti-inflammatory macrophages [8, 9]. In these cells, it is involved in receptor-mediated endocytosis and recycling, intracellular sorting, and transcytosis of altered and normal self-components. More recently, it has been found that the growth and metastases are attenuated in Stab1^{-/-} (Clever-1 knock out) mice in several tumor models, and in mice treated with anti-Clever-1 therapy [10, 11]. In addition, combination treatments with an anti-Clever-1 agent together with an anti-PD-1 agent has shown to produce anti-tumor responses in mouse models of triple negative breast cancer and colorectal cancer [11].

In the publication Viitala, et al (Immunotherapeutic Blockage of Macrophage Clever-1 Reactivates the CD8+ T-cell response against immunosuppressive Tumors, Clinical Cancer Research, American Association for Cancer Research, vol. 25, no. 11, 1 June 2019) the efficacy of immunotherapeutic Clever-1 blockade in combination with anti-PD-1 was tested. It has been observed that combining anti-Clever-1 with anti-PD1 reduces the weight and size of tumours compared to IgG treatment.

The publication WO 2017/182706 discloses a method for estimating efficacy of anti-CLEVER-1 treatment by measuring TNF-alpha secretion and/or HLA-DR expression on peripheral blood monocytes.

### Summary of the Invention

Now, it has been surprisingly found out that anti-Clever-1 treatment in cancer patients decreases PD-1, PD-L1, CTLA-4, OX40, 41BB, LAG3, TIM3 and CD28 expression on leucocytes, especially in circulating T cell populations, and increases the expression of CD25 (IL-2RA), CXCR3 and CD69, and also affecting the expression level of ICOS. In addition, anti-tumor responses with anti-Clever-1 treatment was found to associate with an increase in circulating interferon gamma (IFNg). In line with current knowledge, this means that anti-Clever-1 treatment could later build resistance as the IFNg response could lead to an increase in PD-1 and/or PD-L1 expression. Especially, it has been found that anti-Clever-1 treatment removes T cell exhaustion by downregulating the expression of cell surface markers, known as exhaustion markers or checkpoint molecules, such as PD-1, PD-L1, CTLA-4, ICOS, OX40, 41BB, LAG3, TIM3 CD28, CD25 (IL-2RA), CXCR3 and CD69. By monitoring the change in these commonly known checkpoint molecules during anti-Clever-1 treatment, we can pick a checkpoint inhibitor that should be given in combination with the on-going anti-Clever-1 treatment. If a certain checkpoint molecule does not react to anti-Clever-1 treatment in the wanted way, then a specific checkpoint inhibitor targeting the wanted checkpoint molecule is administered in combination with to the on-going anti-Clever-1 treatment. The expression of cell surface markers can be used to guide anti-Clever-1 treatment or the best possible checkpoint inhibitor(s) combination treatment with anti-Clever-1 therapy. More detailed, it has been found that cell surface markers PD-1, PD-L1, CTLA-4, ICOS, OX40, 41BB, LAG3, TIM3, CD28, CD25 (IL-2RA), CXCR3 and CD69 can be used to monitor patient's response to anti-Clever-1 therapy and to evaluate the need for combination therapy with anti-Clever-1 therapy. The present finding provides a method for choosing the best combination agent(s) to initiate treatment together with anti-Clever-1 therapy after the observed changes in the expression of two, three or more cell surface markers.

Hence, the present invention provides a method for monitoring a patient's response to anti-Clever-1 monotherapy and estimating the need for combination therapy as defined in independent claim 1.

The present invention makes possible a biomarker-based combination treatment for cancer patients and thus reduce or even eliminate the above-mentioned problems in defining non-responders from responding patients. The present invention gives the possibility of probing the molecular landscape of solid tumors via a blood draw to define changes in the patient during anti-Clever-1 therapy and subsequently gives the opportunity to select the best possible check-point inhibition combination treatment with anti-Clever-1 therapy.

The present invention is based on the findings that anti-Clever-1 treatment alternates the expression of several cell surface receptors on leucocytes and that the expression levels of these checkpoints may vary during the treatment course of anti-Clever-1 therapy. This enables the best possible biological rationale to choose which checkpoint inhibitor (ICI) treatment should be combined with anti-Clever-1 treatment and when to initiate that combination treatment during anti-Clever-1 therapy.

The present invention provides means to choose when to start anti-PD-1, anti-PD-L1, anti-CTLA-4, anti-ICOS, anti-OX40, anti-41BB, anti-LAG3, anti-TIM3 and/or anti-CD28 treatment in combination with anti-Clever-1 treatment. Anti-Clever-1 treatment has surprisingly been shown to downregulate exhaustion markers PD-1, PD-L1, CTLA-4, ICOS, OX40, 41BB, LAG3, TIM3 and CD28 in the beginning of the anti-Clever-1 treatment. Thus, for example, if anti-Clever-1 treatment would not downregulate the expression of CTLA-4, one would combine anti-CTLA-4 treatment with anti-Clever-1 treatment. Or if anti-Clever-1 treatment would not downregulate OX40, one would combine anti-OX40 treatment with anti-Clever-1 treatment etc. In similar fashion, despite a significant downregulation of exhaustion markers selected from PD-1, PD-L1, CTLA-4, OX40, 41BB, LAG3, TIM3 and CD28, when beginning anti-Clever-1 treatment, if said exhaustion markers would be upregulated during anti-Clever therapy, then one would combine the given ICI treatment with anti-Clever-1 treatment. Hence, the findings of the present invention can be used to monitor patient's response to anti-Clever-1 treatment during the treatment. For example, despite an initially favorable response to single agent anti-Clever-1 treatment and an increase in serum IFNg levels, T cell activation and an anti-tumor response, the IFNg response could later increase PD-L1 expression, and then it would be best to combine anti-PD-1 and/or anti-PD-L1 treatment with anti-Clever-1 treatment. A method according to an embodiment for monitoring patient's response to anti-Clever-1 therapy comprises measuring and monitoring IFNg levels during anti-Clever-1 treatment.

In a similar fashion, anti-Clever-1 treatment was surprisingly shown to upregulate CD25 (IL-2RA), CXCR3 and CD69 expression in the beginning of the anti-Clever-1 treatment. If this increase would not be seen when initiating anti-Clever-1 treatment, then anti-Clever-1 treatment should be combined with a CD25 (IL-2-RA) or a CXCR3 inducing therapy. In addition, agonists that stimulate CD25 (IL-2-RA) or CXCR3 could be used with anti-Clever-1 treatment to further enhance the proliferation, activation and/or migration of the cells with induced expression of the said molecules.

### Brief Description of the drawings

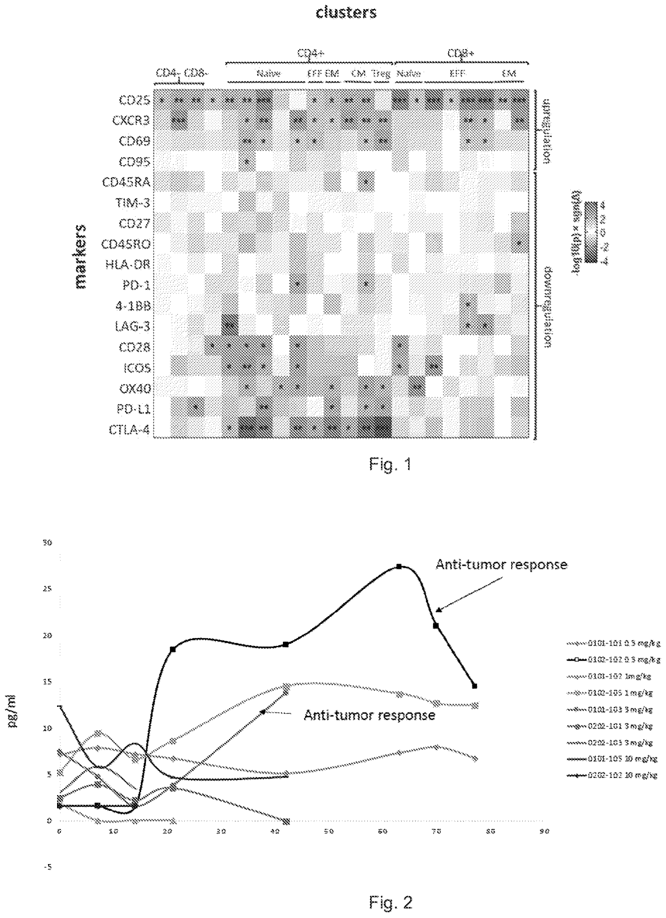

**Figure 1****.** Heat map of changes in cell surface markers (i.e. checkpoints) in seven cancer patients treated with anti-Clever-1 antibody (FP-1305) therapy. The first sample was taken just before dosing FP-1305 and second sample taken 8 days after FP-1305 infusion. The color changes indicate the extent of the average logarithmic change (upregulation or downregulation) in the detection level of the cell surface marker from these seven patients, and the asterisk marks statistical significance of the change pre-dose vs. post-dose from these 7 patients (* p < 0.05, ** p < 0.01, *** p < 0.001).
**Figure 2****.** Change in serum IFNg levels during anti-Clever-1 antibody (FP-1305) treatment. An increase in serum IFNg during FP-1305 treatment is associated with an anti-tumor response in metastatic colorectal cancer, which has not previously responded to any available therapy.

### Detailed description of the invention

CLEVER-1 is a protein disclosed in the patent publication WO 03/057130, Common Lymphatic Endothelial and Vascular Endothelial Receptor-1. It is a binding protein that mediates adhesion of lymphocytes (and malignant tumor cells) to endothelium in both the systemic vasculature and in the lymphatics. By blocking the interaction of Clever-1 and its lymphocyte substrate, it is possible to simultaneously control lymphocyte recirculation and lymphocyte migration, and related conditions such as inflammation, at the site of lymphocyte influx into, and efflux from, the tissues.

The terms "an agent capable of binding to Clever-1", "Clever-1 inhibitor" and "anti-clever-1 agent" are interchangeable and refers to agents including antibodies and fragments thereof, peptides or the like, which are capable of binding to Clever-1 for blocking the interaction of Clever-1 and malignant tumor cells. The agent may also be any other inhibitor, such as small molecule inhibitor or macromolecule having an adequate affinity to bind to Clever-1 receptor and to inhibit the protein activity. The term "an antibody or a fragment thereof' is used in the broadest sense to cover an antibody or a fragment thereof which are capable to bind Clever-1 molecule in an individual. Especially, it shall be understood to include chimeric, humanized or primatized antibodies, as well as antibody fragments and single chain antibodies (e.g. Fab, Fv), so long they exhibit the desired biological activities. Particular useful agents are anti-Clever-1 antibodies and fragments thereof. Therefore, according to an embodiment of the present invention an agent capable of binding to Clever-1, i.e. Clever-1 inhibitor or anti-Clever-1 agent, is selected from the group consisting of an antibody or a fragment thereof, peptide(s), macromolecule and any combination thereof. According to the present invention "anti-Clever-1 treatment" or "anti-Clever-1 therapy" refers to the treatment comprising administration of at least one agent capable of binding Clever-1. Anti-Clever-1 monotherapy refers in the present disclosure to the therapy including anti-Clever-1 agent(s) as a single agent. The claimed invention is limited in this respect to an anti-Clever-1 antibody or a fragment thereof.

According to an embodiment of the invention, an anti-Clever-1 antibody is a therapeutic humanized anti-Clever-1 antibody. According to an embodiment of the present invention, an anti-Clever-1 antibody is a humanized monoclonal Clever-1 antibody, previously presented in the patent publication WO2017/182705.

In an embodiment of the present invention, an anti-Clever-1 antibody is a humanized monoclonal immunoglobulin G4κ antibody bexmarilimab (International Nonproprietary Name (INN)) as disclosed in WHO Drug Information, Vol. 33, No. 4, pages 814-815 (2019)), or bexmarilimab variant or the antibody in a bexmarilimab biosimilar. As used herein, "bexmarilimab" means the IgG4 monoclonal antibody with the structure described in WHO Drug Information, Vol. 33, No. 4, pages 814-815 (2019).

A bexmarilimab biosimilar means a biological product which is approved by a regulatory agency in any country for marketing as a bexmarilimab biosimilar. In an embodiment, a bexmarilimab biosimilar comprises a bexmarilimab variant as the drug substance. In an embodiment, a bexmarilimab biosimilar has substantially the same amino acid sequence of heavy and light chains as bexmarilimab. As used herein, a "bexmarilimab variant" means an antibody which comprises sequences of heavy chain and light chain that are identical to those in bexmarilimab, except for having one or more conservative amino acid substitutions at positions that are located outside of the light chain CDRs and/or one or more conservative amino acid substitutions that are located outside of the heavy chain CDRs, e.g. the variant positions are located in the framework regions or the constant region. In other words, bexmarilimab and a bexmarilimab variant comprise identical CDR sequences, but differ from each other due to having a conservative amino acid substitution at other positions in their full-length light and heavy chain sequences. A bexmarilimab variant is substantially the same as bexmarilimab with respect to binding affinity to CLEVER-1.

According to an embodiment of the present invention, a cell line producing the therapeutic anti-Clever-1 antibody bexmarilimab (FP-1305) has been deposited on 27 May 2020 under the terms of the Budapest Treaty on the International Recognition of the Deposit of Micro-organisms for the Purposes of Patent Procedure with the DSMZ-German Collection of Microorganisms and Cell Cultures GmbH, Inhoffenstrasse 7B, D-38124 Braunschweig, Germany, and has the accession number DSM ACC3361. The present invention is not to be limited in scope by the culture deposited, since the deposited embodiment is intended as a single illustration of one aspect of the invention and any culture that is functionally equivalent is within the scope of this invention. The deposit of material herein does not constitute an admission that the written description herein contained is inadequate to enable the practice of any aspect of the invention, including the best mode thereof, nor is it to be construed as limiting the scope of the claims to the specific illustration that it represents.

It has been observed that anti-Clever-1 treatment has an ability to decrease expression of cell surface markers PD-1, PD-L1, CTLA-4, ICOS, OX40, 41BB, LAG3, TIM3 and CD28 on leucocytes, especially on circulating T cell populations, and an ability to increase the expression of cell surface markers CD25 (IL-2RA), CXCR3 and CD69. However, the effect may vary from patient to other and/or from cancer type to other. For example, cell surface marker ICOS may be upregulate or downregulate in relation to anti-Clever-1 therapy. Therefore for providing most efficient treatment with anti-Clever-1 therapy, the expression of one or more said cell surface marker is monitored when initiating anti-Clever-1 therapy and/or during anti-Clever-1 therapy for providing required information to start the best combination agent(s) treatment together with anti-Clever-1 therapy after the observed changes in the expression of two, three or more cell surface markers.

The present invention provides a method in accordance with independent claim 1.

A sample obtained at a first point in time prior to the administration of an agent capable of binding to Clever-1 to a patient refers to a sample obtained prior to the first administration of an agent capable of binding to Clever-1 (i.e. the first sample). A sample obtained at a later point in time after the administration of an agent capable of binding to Clever-1 to a patient refers to a sample obtained at any time point during the therapy, i.e. after the first administration of an agent capable of binding to Clever-1 to a patient and before the last administration of an agent capable of binding to Clever-1 to a patient.

According to an embodiment of the invention,
- the absence of downregulation of PD-1, PD-L1, CTLA-4, ICOS, OX40, 41BB, LAG3, TIM3 and/or CD28 is an indication for initiation the concomitant administration of an agent that affects said cell surface marker, and/or
- the absence of upregulation of ICOS, CD25 and/or CXCR3 is an indication for initiation the concomitant administration of an agent that affects said cell surface marker, and/or
- the increase of CD25 and/or CXCR3 expression is an indication for initiation the concomitant administration of an agent that affects said cell surface marker.

According to another embodiment of the present invention,
- an upregulation of PD-1, PD-L1, CTLA-4, ICOS, OX40, 41BB, LAG3, TIM3 and/or CD28 in comparison to the expression level of said cell surface marker measured from the sample obtained at a first point of time is an indication for initiation the concomitant administration of an agent that affects said cell surface marker, and/or
- a downregulation of ICOS, CD25 and/or CXCR3 in comparison to the expression level of said cell surface marker measured from the sample obtained at a first point of time is an indication for initiation the concomitant administration of an agent that affects said cell surface marker, and/or
- the increase of CD25 and/or CXCR3 is an indication for initiation the concomitant administration of an agent that affects said cell surface marker.

The expression of two, three or more cell surface marker selected from CTLA-4, ICOS, OX40, 41BB, LAG3, TIM3, CD28, CD25 and CXCR3 is measured from the obtained samples and the expression level of said cell surface marker from the sample obtained at a later point of time is compared to the expression level of said cell surface marker measured from the sample obtained at a first point of time for evaluating for initiation the concomitant administration of an agent that affects said cell surface marker. In an embodiment according to the present invention, an expression of PD-1 and/or PD-L1 can be analyzed in addition to above mentioned cell surface markers for evaluating for initiation the concomitant administration of PD-1/PD-1 inhibitor(s).

According to an embodiment, a method further comprises measuring interferon gamma (IFNg) from the obtained samples and comparing the IFNg level measured from the sample at a later point of time to the IFNg level measured from the sample obtained at a first point of time, wherein an increase in the IFNg level is an indication for initiation the concomitant administration of PD-1 and/or PD-L1 inhibitor.

According to an embodiment of the invention sample is a blood sample drawn from a patient. In the invention, an expression of two, three or more cell surface markers are analyzed in circulating T cells, for example those obtained from a blood sample drawn from the patient.

According to the present invention, the expression levels of the cell surface markers may be measured any suitable method known in the art.

Disclosed but not claimed is a combination of therapeutically effective amounts of:
a) an agent capable of binding to Clever-1, and
b) one or more agent selected from the group comprising, a CTLA-4 inhibitor, an ICOS inhibitor, an ICOS inducer, an OX40 inhibitor, a 41BB inhibitor, a LAG3 inhibitor, a TIM3 inhibitor, a CD28 inhibitor, a cytokine IL-2 including modified versions of it, a CD25 (IL-2RA) agonist, a CXCR3 inducer and a CXCR3 agonist,
are used in a treatment of cancer in an individual which having been treated with anti-Clever-1 monotherapy and said monotherapy does not show a downregulation of cell surface markers CTLA-4, ICOS, OX40, 41BB, LAG3, TIM3 and/or CD28, and/or an upregulation of cell surface markers ICOS, CD25 and/or CXCR3. A combination for use in a treatment of cancer in an individual may comprise one, two, three, four or more agent selected from the group comprising a CTLA-4 inhibitor, an ICOS inhibitor, an ICOS inducer, an OX40 inhibitor, a 41BB inhibitor, a LAG3 inhibitor, a TIM3 inhibitor, a CD28 inhibitor, a cytokine IL-2 including modified versions of it, a CD25 (IL-2RA) agonist, a CXCR3 inducer and a CXCR3 agonist in addition to an agent capable of binding to Clever-1.

It has been noticed that the combination treatment with anti-Clever-1 therapy can be selected on the basis of the measured expressions of the cell surface marker(s). If there is not desired change in the expression level by anti-Clever-1 therapy alone as a single agent, it is an indication to start the administration of an agent affecting said cell surface marker.

According to an embodiment of the present invention, also other cell surface markers can be defined for monitoring the treatment response and evaluating the need for initiating the combination treatment(s) to induce for example CD69, CD95, CD45RO and/or HLA DR expression.

Disclosed but not claimed is a combination for use in a treatment of cancer in an individual which may further comprise a PD-1 and/or PD-L1 inhibitor, when anti-Clever-1 monotherapy does not show a downregulation of PD-1 and/or PD-L1. A combination for use in a treatment of cancer in an individual may comprise one, two, three, four or more agent selected from the group comprising a CTLA-4 inhibitor, an ICOS inhibitor or inducer, an OX40 inhibitor, a 41BB inhibitor, a LAG3 inhibitor, a TIM3 inhibitor, a CD28 inhibitor, a cytokine IL-2 including modified versions of it or CD25 (IL-2RA) agonist and a CXCR3 inducer or CXCR3 agonist in addition to an agent capable of binding to Clever-1 and PD-1 and/or PD-L1. Again, combinations for use are not claimed as such. The same applies to corresponding methods of treatment.

Disclosed but not claimed is a combination for use in a treatment of cancer in an individual which further comprises a PD-1 and/or PD-L1 inhibitor, when an upregulation of interferon gamma (IFNg) and/or an upregulation of PD-1 and/or PD-L1 is observed during anti-Clever-1 therapy in combination with one or more said agents affecting cell surface marker(s). It has been observed that anti-tumor responses with anti-Clever-1 treatment associates with an increase in circulating interferon gamma (IFNg), which would later lead to an increase in PD-1 and/or PD-L1 expression level. Hence, when increased expression level of IFNg and/or increased expression level of PD-1 and/or PD-L1 is observed during anti-Clever-1 therapy alone or in combination with one or more said agents affecting cell surface marker(s), it is an indication to initiate anti-PD-1 and/or PD-L1 therapy with anti-Clever-1 treatment.

Disclosed but not claimed is a combination for use in a treatment of cancer in an individual which may comprise a combination of a PD-1 and/or PD-L1 inhibitor and an agent capable of binding to Clever-1, when anti-Clever-1 monotherapy does not show a downregulation of PD-1 and/or PD-L1 on lymphocytes and/or an upregulation of IFNg and/or an upregulation of PD-1 and/or PD-L1 is observed during anti-Clever-1 therapy.

CTLA-4 inhibitor, ICOS inhibitor, ICOS inducer, OX40 inhibitor, 41BB inhibitor, LAG3 inhibitor, TIM3 inhibitor, CD28 inhibitor, PD-1 inhibitor and PD-L1 inhibitor comprises an antibody or fragment thereof capable of blocking said cell surface receptors. A combination treatment can comprise any known agent(s) capable of blocking said cell surface receptors.

A cytokine IL-2 including modified versions of it, CD25 (IL-2RA) agonist, a CXCR3 inducer and a CXCR3 agonist comprises an agent capable of activating said cell surface receptor. IL-2 is pro-inflammatory cytokine known to stimulate the immune system (mainly T cells) to kill tumor cells. IL-2 therapies are, however, rather toxic and many developmental technologies are being explored to induce IL-2 in the tumor microenvironment without toxic side effect. Such technologies include IL-2 receptor agonist, cancer vaccines introducing IL-2 producing viruses etc. A combination treatment can comprise any known agent(s) capable of activating said cell surface receptors.

Disclosed but not claimed is a combination for use in a treatment of cancer in an individual which comprises effective amounts of
a) an agent capable of binding to Clever-1, such as anti-Clever-1 antibody or fragment thereof, and
b) one or more of the following agents:
   - a CTLA-4 inhibitor, such as anti-CTLA-4 antibody or fragment thereof that binds specifically to a CTLA-4 receptor and inhibits CTLA-4 activity, when anti-Clever-1 single agent activity does not lead to decreased CTLA-4 expression,
   - an ICOS inhibitor and/or an ICOS inducer, such as anti-ICOS antibody or fragment thereof that binds specifically to an ICOS receptor, when anti-Clever-1 single agent activity does not lead to change in ICOS expression,
   - an OX40 inhibitor, such as anti-OX40 antibody or fragment thereof that binds specifically to an OX40 receptor and inhibits OX40 activity, when anti-Clever-1 single agent activity does not lead to decreased OX40 expression,
   - a 41BB inhibitor, such as anti-41BB antibody or fragment thereof that binds specifically to a 41BB receptor and inhibits 41BB activity, when anti-Clever-1 single agent activity does not lead to decreased 41BB expression,
   - a LAG3 inhibitor, such as anti-LAG3 antibody or fragment thereof that binds specifically to a LAG3 receptor and inhibits LAG3 activity, when anti-Clever-1 single agent activity does not lead to decreased LAG3 expression,
   - a TIM3 inhibitor, such as anti-TIM3 antibody or fragment thereof that binds specifically to a TIM3 receptor and inhibits TIM3 activity, when anti-Clever-1 single agent activity does not lead to decreased TIM3 expression,
   - a CD28 inhibitor, such as anti-CD28 antibody or fragment thereof that binds specifically to a CD28 receptor and inhibits CD28 activity, when anti-Clever-1 single agent activity does not lead to decreased CD28 expression,
   - a cytokine IL-2 including modified versions of it, if the said molecule is used to stimulate the immune system in combination with anti-Clever-1,
   - a CD25 agonist, if anti-Clever-1 single agent activity does not lead to increased CD25 expression,
   - a CXCR3 inducer, if anti-Clever-1 single agent activity does not lead to increased CXCR3 expression,
   - a CXCR3 agonist, if the said molecule is used to stimulate the immune system in combination with anti-Clever-1.

Disclosed but not claimed is a combination for use in a treatment of cancer in an individual which further comprises therapeutically effective amounts of a PD-1 and/or PD-L1 inhibitor, such as anti-PD-1 and/or anti-PD-L1 antibody or fragment thereof that binds specifically to a Programmed Death-1 (PD-1) receptor and inhibits PD-1 activity, when anti-Clever-1 single agent activity does not lead to decreased PD-1/PD-L1 expression and/or an IFNg response lead to increased levels of PD-1/PD-L1 expression.

Disclosed but not claimed is a method for treating a cancer patient comprises administering to said cancer patient a combination of
a) an agent capable of binding to Clever-1, and
b) one or more agent selected from the group comprising a CTLA-4 inhibitor, an ICOS inhibitor, an ICOS inducer, an OX40 inhibitor, a 41BB inhibitor, a LAG3 inhibitor, a TIM3 inhibitor, a CD28 inhibitor, a cytokine IL-2 including modified versions of it, a CD25 (IL-2RA) agonist, a CXCR3 inducer and a CXCR3 agonist.

Disclosed but not claimed is a method for treating a cancer patient comprises administering to said cancer patient a combination of
a) an agent capable of binding to Clever-1, and
b) one or more agent selected from the group comprising a CTLA-4 inhibitor, an ICOS inhibitor, an ICOS inducer, an OX40 inhibitor, a 41BB inhibitor, a LAG3 inhibitor, a TIM3 inhibitor, a CD28 inhibitor, a cytokine IL-2 including modified versions of it, a CD25 (IL-2RA) agonist, a CXCR3 inducer and a CXCR3 agonist, and
c) a PD-1 inhibitor and/or PD-L1 inhibitor.

Disclosed but not claimed is a method for treating a cancer patient comprises administering to said cancer patient a combination of
a) an agent capable of binding to Clever-1, and
b) a PD-1 inhibitor and/or PD-L1 inhibitor,
when anti-Clever-1 monotherapy does not show a downregulation of PD-1 and/or PD-L1 on leucocytes, and/or an upregulation of IFNg and/or PD-1 and/or PD-L1 is observed during anti-Clever-1 therapy.

The term "treatment" or "treating" shall be understood to include complete curing of a disease or disorder, as well as amelioration or alleviation of said disease or disorder. The term "therapeutically effective amount" is meant to include any amount of an agent according to the present invention that is sufficient to bring about a desired therapeutic result.

A method and a composition for treating cancer by reducing malignant tumor growth and/or by inhibiting metastasis formation is applicable to all forms of cancers. Thus, any malignant tumor or metastasis can be treated.

"Administering" refers to the physical introduction of a composition comprising said therapeutic agents to an individual, using any of the various methods and delivery systems known to those skilled in the art. The agents to be used may be administered by any means that achieve their intended purpose. For example, administration may be intravenous, intramuscular, intraperitoneal, intra-tumoral, subcutaneous or other parenteral routes of administration, for example by injection. In addition to the pharmacologically active compounds, the pharmaceutical preparations of said agents preferably contain suitable pharmaceutically acceptable carriers comprising excipients and auxiliaries that facilitate processing of the active agents into preparations that can be used pharmaceutically. The dose chosen should be sufficient to reduce or inhibit malignant tumor growth and/or inhibit metastasis formation.

Disclosed but not claimed is a method for treating a cancer patient comprising
- measuring the expression of two, three or more cell surface marker selected from PD-1 or PD-L1, CTLA-4, ICOS, OX40, 41BB, LAG3, TIM3, CD28, CD25 and CXCR3from the sample obtained from the patient,
- administering an agent capable of binding to Clever-1 to a patient,
- measuring the expression of two, three or more cell surface marker selected from PD-1 or PD-L1, CTLA-4, ICOS, OX40, 41BB, LAG3, TIM3, CD28, CD25 and CXCR3, from the sample obtained from the patient after administration of an agent capable of binding to Clever-1 to a patient,
- comparing the expression level of said cell surface marker measured from the sample obtained at a later point of time to the expression level of said cell surface marker measured from the sample obtained at a previous point of time, wherein the absence of the desired change in the expression level of the cell surface marker is an indication for initiation the concomitant administration of an agent that affects said cell surface marker, and
- administering one or more agent that affects said cell surface marker with observed absence of the desired change in expression level.

### EXPERIMENTAL

An anti-Clever-1 antibody FP-1305 (DSM ACC3361) used in the following Examples is a humanized antibody currently being developed by Faron Pharmaceuticals for cancer treatment and it is disclosed more detailed in the patent publication WO2017/182705 and known as bexmarilimab.

### Example 1. Cell surface/exhaustion marker changes in leucocyte populations of cancer patients that have started anti-Clever-1 (FP-1305) treatment

Clever-1 inhibiting agent, an anti-Clever-1 antibody FP-1305 is currently being tested for safety and preliminary efficacy in a Phase I/II study in patient with advanced solid tumors (clinicaltrials.gov NCT03733990: A Study to Evaluate Safety, Tolerability and Preliminary Efficacy of FP-1305 in Cancer Patients (MATINS)). First (pre-dose) sample taken prior to initiating FP-1305. Second sample (post-dose) taken 8 days after beginning FP-1305 treatment. Cell surface markers are analyzed from the samples as described below.

### Protocol for Mass Cytometry (CyTOF) staining and data analysis

PBMCs were isolated from the samples with Ficoll-Paque density gradient centrifugation and frozen with 10% DMSO in RPMI1640 (Sigma-Aldrich; RPMI1640 supplemented with 10% FCS, 2 mmol/L L-glutamine) medium.

Prior to CyTOF staining frozen PBMCs were thawn and re-suspended in PBS counted. 1-3×10⁶ cells were taken for stainings. First cells were stained with 2,5 µM Cell-ID cisplatin (Fluidigm; cat. 201064) viability reagent for 5 min at room temperature (RT). After washings, cells were barcoded with heavy-metal isotope-labelled anti-human CD45 (clone H130) antibodies (CD45_89Y, CD45_141Pr and CD45_147Sm, 1/200) for 30 min at RT, washed carefully and differentially barcoded samples were combined. Next, samples were blocked with human Kiovig solution (0,2 mg/ml) for 15 min at RT and stained with heavy-metal isotope-labelled anti-human antibody cocktail (Table 1) including cell surface markers for 30 min at RT, followed with washings.

**Table1. Antibodies used in CyTOF staining PANEL.**

| **Tag** | **Marker** | **Clone** |
|---|---|---|
| 154Sm | CD3 | UCHT1 |
| 145Nd | CD4 | RPA-T4 |
| 146Nd | CD8a | RPA-T8 |
| 143Nd | CD127 | A019D5 |
| 167Er | CD27 | O323 |
| 165Ho | CD45RO | UCHL1 |
| 169Tm | CD45RA | HI100 |
| 170Er | HLA-DR | L243 |
| 149Sm | CD25 | 2A3 |
| 163Dy | CXCR3 | G025H7 |
| 160Gd | CD28 | CD28.2 |
| 176Yb | CD196/CCR6 | G034E3 |
| 144Nd | CD69 | FN50 |
| 155Gd | CD279/PD-1 | EH12.2H7 |
| 159Tb | CD274/PD-L1 | 29E.2A3 |
| 161Dy | CTLA-4 | 14D3 |
| 158Gd | OX40 | ACT35 |
| 175Lu | LAG-3 | 11C3C65 |
| 153Eu | TIM-3 | F38-2E2 |
| 173Yb | CD137/4-1BB | 4B4-1 |
| 152Sm | CD95 | DX2 |
| 168Er | CD278/ICOS | C398.4A |
| 166Er | CD197/CCR7 | G043H7 |

Stained samples were incubated with DNA intercalation reagent (1/1000, Cell ID Intercalator-103Rh in MaxPar^{®} Fix and Perm Buffer; cat. 201067; Fluidigm) for 1h at RT, washed and fixed with 4% PFA solution overnight (o/n) at +4°C. Next day samples were washed, resuspended to MaxPar Water (cat. 201069; Fluidigm) containing a 1/10 dilution of EQ 4 Element Beads (Fluidigm) and immediately acquired to a CyTOF mass cytometer (Helios, Fluidigm). After the bead normalization of the samples, viable singlet cells were debarcoded by using FlowJo. CD45+CD3+ cells were gated and exported for further analysis.

The data analysis was performed similarly as in Kimball et al 2019 J Immunol (A Beginner's Guide to Analyzing and Visualizing Mass Cytometry Data). R studio version 1.2.1335 was downloaded from the official R Web site and Cytokit package was downloaded from Bioconductor and opened in R. Manually gated events (gated as explained above) were imported into Cytokit and subjected to Phenograph analysis. Clustering was performed by using 9 out of 23 markers (CD4, CD8, CD45RA, CCR7, CD45RO, CD127, CD25, CCR6, CXCR3) with additional settings: merge method; minimum, transformation; CytofAsinh, cluster method; Rphenograph, visualization method; tSNE and cellular progression NULL.

22 clusters were defined by Phenograph and these clusters were displayed on tSNE blots by using R package "shiny" to visualize different patients before and after treatment. Cluster colours, identification numbers, dot and label size was customised in the "shiny" app. The phenograph analysis produced several csv-files which were used to calculate mean expression values per sample for each marker and additionally the changes between pre and post samples per patient was calculated. Heatmap was generated with ComplexHeatmap package ("Gu, Z. (2016) Complex heatmaps reveal patterns and correlations in multidimensional genomic data. Bioinformatics.") downloaded from Bioconductor and statistical analysis was done with R version 3.6.1 ("R Core Team (2019). R: A language and environment for statistical computing. R Foundation for Statistical Computing, Vienna, Austria. URL https://www.R-project.org/.") by using T-test.

The results are shown in Figure 1: the difference of marker expression between pre and post (day8) samples in each cluster.

### Example 2. Serum IFNg increase is associated with an anti-tumor response in cancer patients treated with anti-Clever-1 (FP-1305)

The anti-Clever-1 antibody FP-1305 has begun clinical development in the setting explained above. In this first-in human trial (clinicaltrials.gov NCT03733990) metastatic colorectal cancer patients that have not been responsive to any available therapy have shown anti-tumor responses. These so far have all been associated with an increase in serum IFNg levels during treatment (Fig 2). IFNg serum levels were measured using multiplex cytokine panel at the beginning of every treatment cycle. The treatment cycle, i.e. interval between every FP-1305 infusion was 3 weeks. It is current knowledge that elevated levels of IFNg may lead to elevated levels of PD-L1 and resistance to T cell targeted tumor killing. If patients receiving anti-Clever-1 treatment would later on face resistance due to increased levels of IFNg and following PD-L1, then these patients would need anti-PD-1/L1 therapy with anti-Clever-1 treatment.

### Cited references:

[1] Pardoll DM., The blockade of immune checkpoints in cancer immunotherapy, Nature reviews cancer, 2012, 12(4): 252 - 264).
[2] Chen DS, Mellman I. Elements of cancer immunity and the cancerimmune set point. Nature 2017; 541(7637): 321-30.
[3] Hegde PS, Karanikas V, Evers S. The Where, the When, and the How of Immune Monitoring for Cancer Immunotherapies in the Era of Checkpoint Inhibition. Clin Cancer Res 2016;22(8):1865-74 doi 10.1158/1078-0432.CCR-15-1507.
[4] Guerriero JL, Sotayo A, Ponichtera HE, Castrillon JA, Pourzia AL, Schad S, et al. Class IIa HDAC inhibition reduces breast tumours and metastases through anti-tumour macrophages. Nature 2017; 543(7645):428-32.
[5] Qian BZ, Pollard JW. Macrophage diversity enhances tumor progression and metastasis. Cell 2010; 141(1):39-51.
[6] Ries CH, Cannarile MA, Hoves S, Benz J, Wartha K, Runza V, et al. Targeting tumor-associated macrophages with anti-CSF-1R antibody reveals a strategy for cancer therapy. Cancer Cell 2014; 25(6):846-59.
[7] Quail DF, Bowman RL, Akkari L, Quick ML, Schuhmacher AJ, Huse JT, et al. The tumor microenvironment underlies acquired resistance to CSF-1R inhibition in gliomas. Science 2016;352(6288): aad3018.
[8] Kzhyshkowska J, Gratchev A, Goerdt S. Stabilin-1, a homeostatic scavenger receptor with multiple functions. J Cell Mol Med 2006;10(3):635-49.
[9] Kzhyshkowska J, Workman G, Cardó-Vila M, Arap W, Pasqualini R, Gratchev A, et al. Novel function of alternatively activated macrophages: stabilin-1-mediated clearance of SPARC. J Immunol 2006;176(10):5825-32.
[10] Karikoski M, Marttila-Ichihara F, Elima K, Rantakari P, Hollmen M, Kelkka T, et al. Clever-1/Stabilin-1 Controls Cancer Growth and Metastasis. Clinical Cancer Research 2014;20(24):6452-64.
[11] Viitala M., Virtakoivu R., Tadauon S., Rannikko J., Jalkanen S., Hollmén M., Immunotherapeutic Blockade of Macrophage Clever-1 Reactivates the CD8+ T-cell Response against Immunosuppressive Tumors. Clin Canc Res. Feb 2019 doi: 10.1158/1078-0432

## Claims

1. A method for monitoring a cancer patient's response to anti-Clever-1 therapy and evaluating the need for combination therapy, when an agent capable of binding to Clever-1 has been administered in the patient, the method comprising
- providing an obtained sample from patient at a first point in time prior to the administration of an agent capable of binding to Clever-1 to a patient,
- providing an obtained sample from patient at a later point in time after the administration of an agent capable of binding to Clever-1 to a patient,
- measuring the expression of two, three or more cell surface markers selected from PD-1 or PD-L1, CTLA-4, ICOS, OX40, 41BB, LAG3, TIM3, CD28, CD25 and CXCR3 from the obtained samples, and
- comparing the expression level of said cell surface markers measured from the sample obtained at a later point of time to the expression level of said cell surface markers measured from the sample obtained at a first point of time, wherein the absence of the desired change in the expression level of a cell surface marker is an indication for initiation the concomitant administration of an agent that affects said cell surface marker,
wherein the markers are measured in circulating T cells, and
wherein the agent capable of binding to Clever-1 comprises an anti-Clever-1 antibody or a fragment thereof.

2. The method according to claim 1, **characterized in that** the sample comprises circulating T cells obtained from a blood sample drawn from the patient.

3. The method according to claim 1 or 2, **characterized in that** anti-Clever-1 antibody is bexmarilimab.

4. The method according to claim 3, **characterized in that** anti-Clever-1 antibody is anti-Clever-1 antibody FP-1305 (DSM ACC3361).

## Patentansprüche

1. Ein Verfahren zur Überwachung des Ansprechens eines Krebspatienten auf eine anti-Clever-1-Therapie und zur Bewertung der Notwendigkeit einer Kombinationstherapie, wenn ein Mittel, das an Clever-1 binden kann, an den Patienten verabreicht wurde, wobei das Verfahren Folgendes umfasst
- Bereitstellung einer vom Patienten gewonnenen Probe zu einem ersten Zeitpunkt vor der Verabreichung eines Mittels, das an Clever-1 binden kann, an den Patienten,
- die Bereitstellung einer vom Patienten gewonnenen Probe zu einem späteren Zeitpunkt nach der Verabreichung eines Mittels, das an Clever-1 binden kann, an einen Patienten,
- Messen der Expression von zwei, drei oder mehr Zelloberflächenmarkern, ausgewählt aus PD-1 oder PD-L1, CTLA-4, ICOS, OX40, 41BB, LAG3, TIM3, CD28, CD25 und CXCR3 aus den erhaltenen Proben, und
- Vergleichen des Expressionsniveaus der genannten Zelloberflächenmarker, das von der zu einem späteren Zeitpunkt erhaltenen Probe gemessen wurde, mit dem Expressionsniveau der genannten Zelloberflächenmarker, das von der zu einem ersten Zeitpunkt erhaltenen Probe gemessen wurde, wobei das Fehlen der gewünschten Änderung des Expressionsniveaus eines Zelloberflächenmarkers ein Anzeichen für die Einleitung der begleitenden Verabreichung eines Mittels ist, das den genannten Zelloberflächenmarker beeinflusst,
wobei die Marker in zirkulierenden T-Zellen gemessen werden und
wobei das Mittel, das in der Lage ist, an Clever-1 zu binden, einen Anti-Clever-1-Antikörper oder ein Fragment davon umfasst.

2. Das Verfahren nach Anspruch 1, **dadurch gekennzeichnet, dass** die Probe zirkulierende T-Zellen umfasst, die aus einer dem Patienten entnommenen Blutprobe gewonnen wurden.

3. Das Verfahren nach Anspruch 1 oder 2, **dadurch gekennzeichnet, dass** der Anti-Clever-1-Antikörper Bexmarilimab ist.

4. Das Verfahren nach Anspruch 3, **dadurch gekennzeichnet, dass** der Anti-Clever-1-Antikörper der Anti-Clever-1-Antikörper FP-1305 (DSM ACC3361) ist.

## Revendications

1. Procédé de surveillance de la réponse d'un patient atteint de cancer à une thérapie anti-Clever-1 et d'évaluation de la nécessité d'une thérapie combinée, lorsqu'un agent capable de se lier à Clever-1 a été administré au patient, le procédé comprenant
- la fourniture d'un échantillon obtenu auprès d'un patient à un premier moment avant l'administration d'un agent capable de se lier à Clever-1 à un patient,
- la fourniture d'un échantillon obtenu auprès d'un patient à un moment ultérieur après l'administration d'un agent capable de se lier à Clever-1 à un patient,
- la mesure de l'expression de deux, trois ou plusieurs marqueurs de surface cellulaire choisis parmi PD-1 ou PD-L1, CTLA-4, ICOS, OX40, 41BB, LAG3, TIM3, CD28, CD25 et CXCR3 à partir des échantillons obtenus, et
- la comparaison du niveau d'expression desdits marqueurs de surface cellulaire mesuré à partir de l'échantillon obtenu à un moment ultérieur au niveau d'expression desdits marqueurs de surface cellulaire mesuré à partir de l'échantillon obtenu à un premier moment, dans lequel l'absence du changement souhaité dans le niveau d'expression d'un marqueur de surface cellulaire est une indication pour l'initiation de l'administration concomitante d'un agent qui affecte ledit marqueur de surface cellulaire,
dans lequel les marqueurs sont mesurés dans des lymphocytes T circulants, et
dans lequel l'agent capable de se lier à Clever-1 comprend un anticorps anti-Clever-1 ou un fragment de celui-ci.

2. Procédé selon la revendication 1, **caractérisé en ce que** l'échantillon comprend des lymphocytes T circulants obtenus à partir d'un échantillon de sang prélevé sur le patient.

3. Procédé selon la revendication 1 ou 2, **caractérisé en ce que** l'anticorps anti-Clever-1 est le bexmarilimab.

4. Procédé selon la revendication 3, **caractérisé en ce que** l'anticorps anti-Clever-1 est l'anticorps anti-Clever-1 FP-1305 (DSM ACC3361).
